# EUROPEAN PATENT APPLICATION

(11) **EP 1 197 489 A1**
(43) Date of publication of application: **17.04.2002**
(21) Application number: 00907996.3
(22) Date of filing: 10.03.2000
(51) Int. Cl.: C07D 239/32, C07D 239/47

(54) **PYRIMIDINE DERIVATIVES AND PROCESS FOR THE PREPARATION THEREOF**

(30) Priority: 12.03.1999 JP 6606499
(71) Applicant: ONO PHARMACEUTICAL CO., LTD., Osaka-shi, Osaka 541-8526 (JP)
(72) Inventor: KOJIMA, T., Fukui Res. Inst., ONO Pharm Co. Ltd, Sakai-gun, Fukui 913-0032 (JP); HACHIYA, K., Fukui Res. Inst., ONO Pharm Co. Ltd, Sakai-gun, Fukui 913-0032 (JP); OHMOTO, K, Minase Res. Inst., ONO Pharm Co. Ltd, Osaka 618-8585 (JP)
(74) Representative: Henkel, Feiler, Hänzel
(86) International application number: JP0001465
(87) International publication number: WO0055140

(57) **Abstract**

Novel pyrimidine derivatives represented by formula (I): 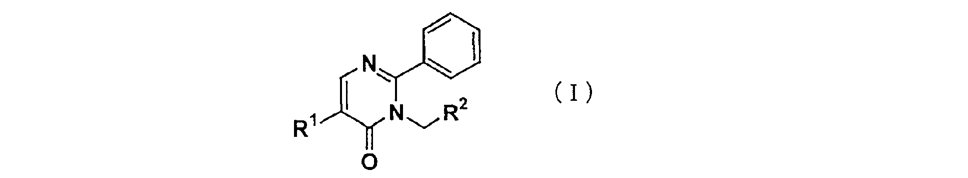 (wherein R¹ represents a 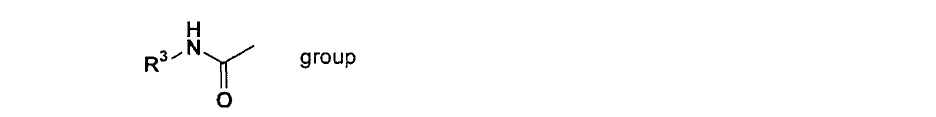 (in the group, R³ represents a leaving group), a hydroxycarbamoyl group, or an amino group; and R² represents a group capable of converting into a formyl group), and a process for producing a compound represented by formula (II): 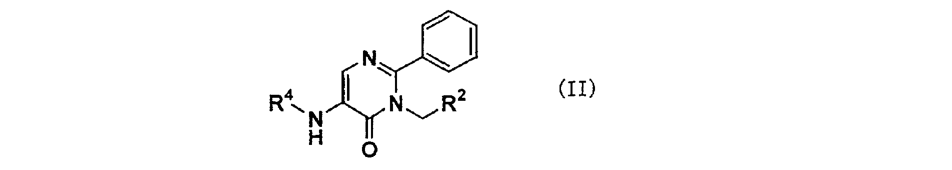 (wherein R⁴ represents a protective group of an amino group; and R² has the same meaning as described above) using the derivative.

According to the invention, a compound represented by formula (II) useful as an intermediate for pharmaceuticals can be produced industrially and efficiently via a novel intermediate represented by formula (I).

## Description

### TECHNICAL FIELD

The invention relates to pyrimidine derivatives useful as intermediates for producing pharmaceuticals, a process for producing the same, and a process for producing pyrimidine derivatives using the intermediate.

More specifically, the invention relates to:
(1) a compound represented by formula (I): 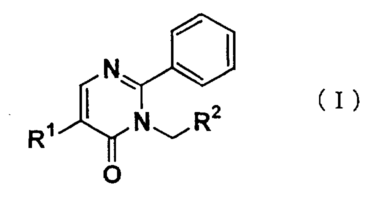 (wherein all symbols have the same meanings as described below),
(2) a process for producing the same, and
(3) a process for producing a compound represented by formula (II): 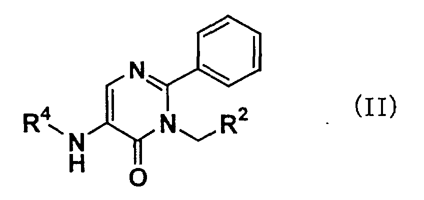 (wherein all symbols have the same meanings as described below), comprising using the compound represented by formula (1).

### BACKGROUND ART

The compound represented by formula (A): 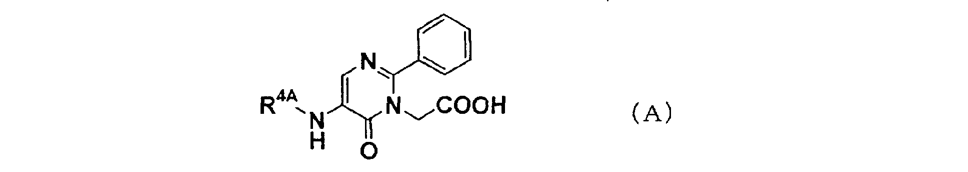 (wherein R^{4A} represents an amino-protective group), particularly the compound represented by formula (A') wherein R^{4A} represents a benzyloxycarbonyl group: 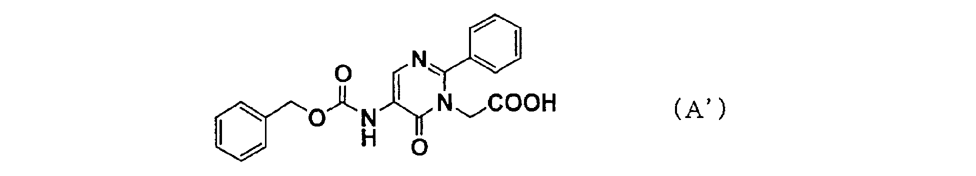 is disclosed as:
(1) an intermediate for producing an elastase inhibitor in EP528633 (JP-A-5-286946), WO9321210 (JP-A-7-505876), WO9321214 (JP-A-7-505877) and *J. Med. Chem.,* 38, 98-108 (1995),
(2) an intermediate for producing a serine protease inhibitor in WO9824806,
(3) an intermediate for producing a chymase inhibitor in WO9633974 and WO9809949, and
(4) an intermediate for producing an interleukin-1β converting enzyme inhibitor in W09526958 (JP-A-9-511249) and J. *Med. Chem.,* 39, 2438-2440 (1996).

From the above, the compound represented by formula (A) is a very important compound as an intermediate for producing a medicament.

On the other hand, the above EP528633 (JP-A-5-286946) discloses a process shown in the following reaction scheme 1 as a process for producing the compound represented by formula (A').

In the reaction scheme 1, R^{8A} represents a dimethoxymethyl group, a diethoxymethyl group, or a vinyl group; Bn represents a benzyl group; Et₃N represents triethylamine; and DPPA represents diphenylphosphoryl azide.

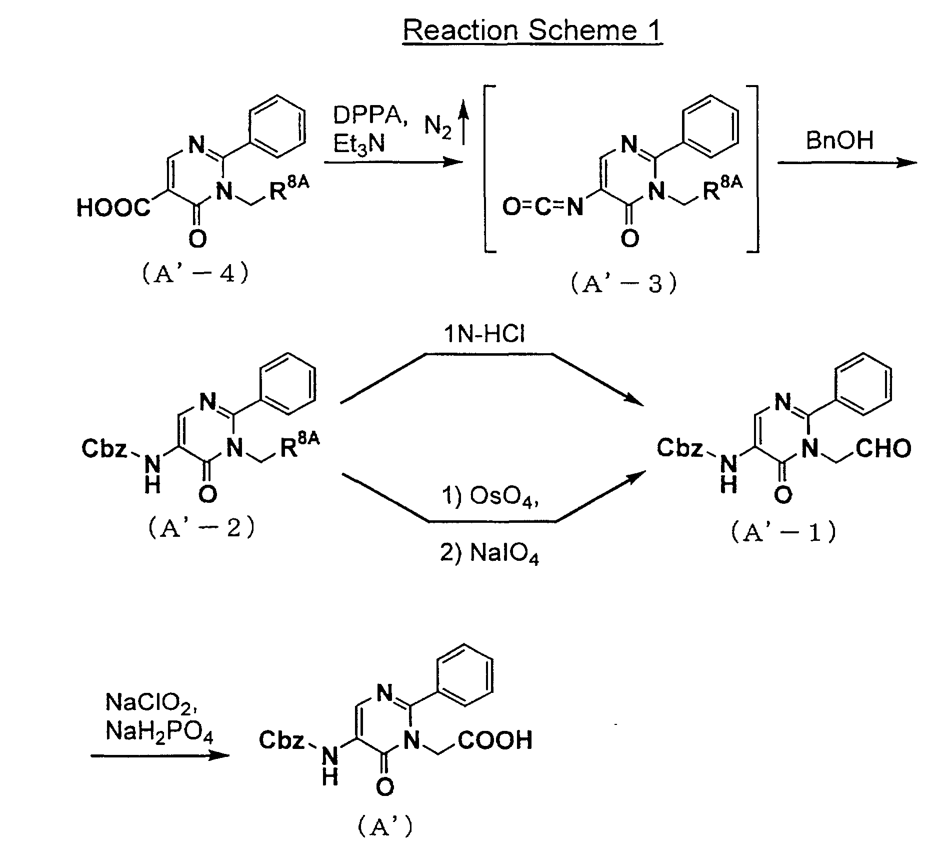

Moreover, in the above specification, it is described that the compound wherein R^{4A} represents a group other than a benzyloxycarbonyl group is also produced in a similar manner to the process shown in Reaction scheme 1.

In the conventional process shown in Reaction scheme 1, the Curtius rearrangement is carried out for producing a compound represented by formula (A'-2) (a compound wherein R² represents a dimethoxymethyl group, a diethoxymethyl group, or a vinyl group; and R⁴ represents a benzyloxycarbonyl group in formula (II) in the invention) from a compound represented by formula (A'-4). However, the reaction results in no problem at a small scale synthesis, but at a large scale synthesis, there exists danger of explosion owing to a large scale formation of nitrogen gas at the reaction.

Moreover, it is necessary to use benzyl alcohol in introducing a benzyloxycarbonyl group such as the compound represented by formula (A'-2), but it is very difficult to remove unreacted benzyl alcohol in the purification process after the completion of the reaction.

As a result of extensive studies for overcoming the above problems, the present inventors have found a novel process for producing a compound of formula (II) shown in Reaction scheme 2 via novel intermediate compounds represented by formulae (I-1), (I-2) and (I-3), i.e., compounds represented by formula (I).

In Reaction Scheme 2, R² represents a group capable of converting into a formyl group; R³ represents a leaving group; and R⁴ represents an amino-protective group.

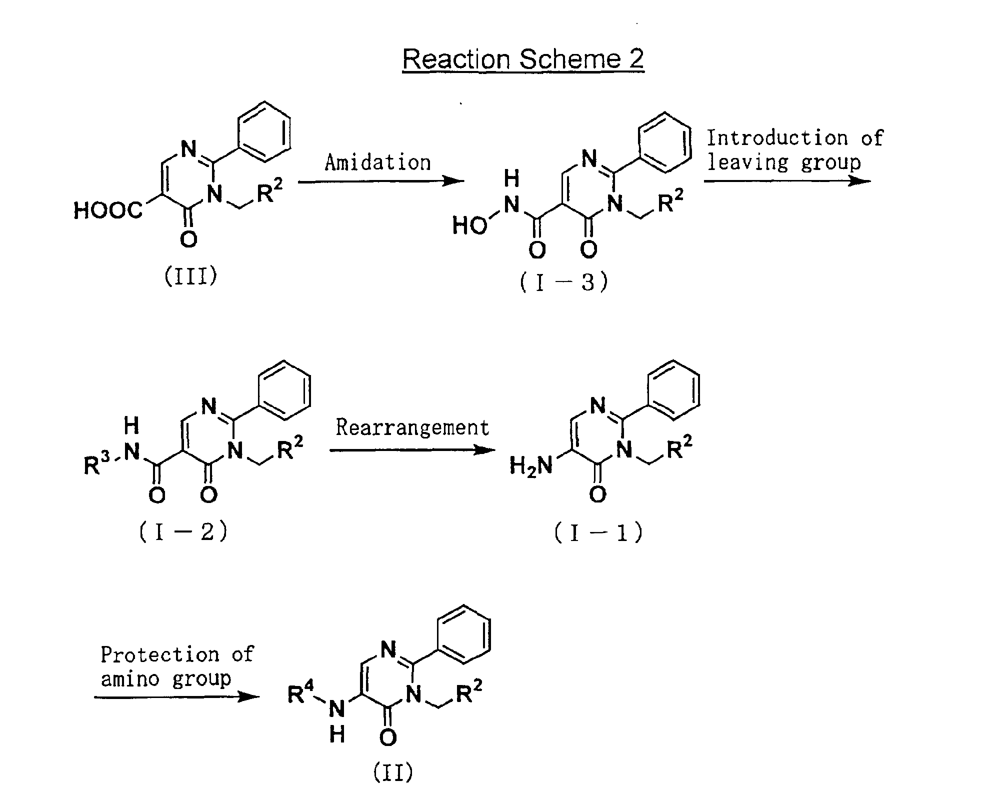

The present inventors have found that the known compound represented by formula (II) (described in EP528633) can be produced according to the process shown in Reaction scheme 2 without carrying out the Curtius rearrangement which is dangerous at a large scale synthesis, and have accomplished the invention.

### DISCLOSURE OF THE INVENTION

The invention relates to
1) a compound represented by formula (I): 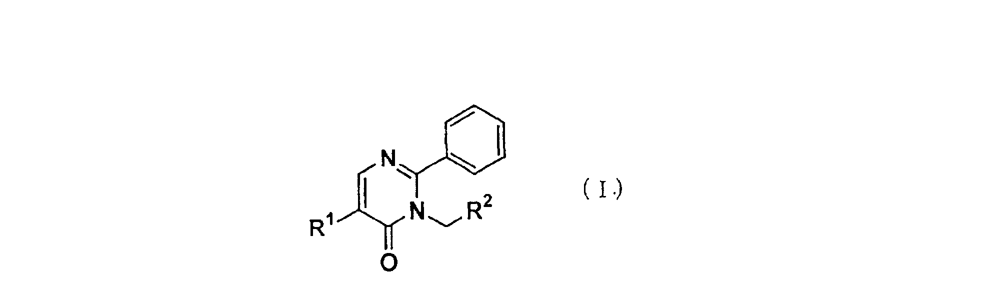 (wherein R¹ represents a 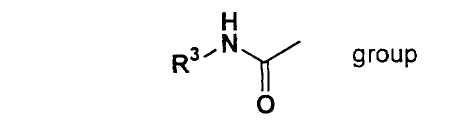 (in the group, R³ represents a leaving group), a hydroxycarbamoyl group, or an amino group; and R² represents a group capable of converting into a formyl group),
2) a process for producing the compound represented by formula (I), and
3) a process for producing a compound represented by formula (II): 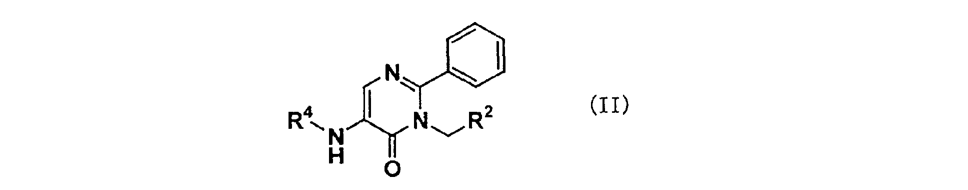 (wherein R⁴ represents an amino-protective group, and other symbols have the same meanings as described above), comprising using the compound represented by formula (I).

### DETAILED DESCRIPTION OF THE INVENTION

In the compounds represented by formulae (I) and (II), examples of the group capable of converting into a formyl group represented by R² include a dimethoxymethyl group, a diethoxymethyl group, a vinyl group, and the like, but the group is not particularly limited, so long as it is a group capable of converting into a formyl group.

As the group capable of converting into a formyl group represented by R², a dimethoxymethyl group, a diethoxymethyl group, and a vinyl group are preferred, and a dimethoxymethyl group is more preferred.

Examples of the leaving group represented by R³ include carbonyloxy derivatives (e.g., acetyloxy group, pivaloyloxy group, benzoyloxy group, *etc*.), sulfonyloxy group (e.g., mesyl group, tosyl group, *etc*.), and the like.

As the leaving group represented by R³, an acetyloxy group and a pivaloyloxy group are preferred, and an acetyloxy group is more preferred.

In the compound represented by formula (II), the amino-protective group represented by R⁴ includes a benzyloxycarbonyl group, a methoxycarbonyl group, a t-butoxycarbonyl group, a trifluoroacetyl group, and the like, but is not particularly limited thereto, so long as it is a group which is easily and selectively removable. For example, those described in T. W. Greene, *Protective Groups in Organic Synthesis,* Wiley, New York, 1991 can be employed.

As the amino-protective group represented by R⁴, a benzyloxycarbonyl group is preferred.

### PROCESS FOR PRODUCING THE COMPOUNDS OF THE INVENTION

The compound of the invention represented by formula (I) can be produced according to processes (1) to (3) described below, the processes described in the following examples, or known processes.
(1) Among the compounds represented by formula (I), the compound wherein R¹ represents a hydroxycarbamoyl group, i.e., the compound represented by formula (I-3): 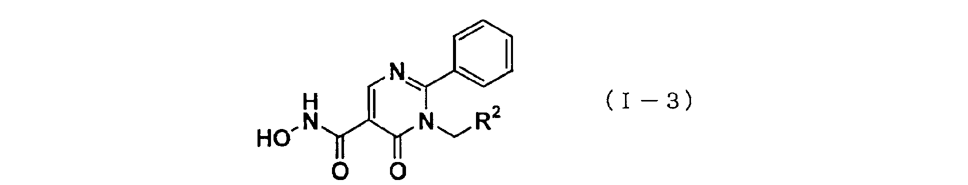 (wherein R² represents a group capable of converting into a formyl group) can be produced by subjecting the compound represented by formula (III): 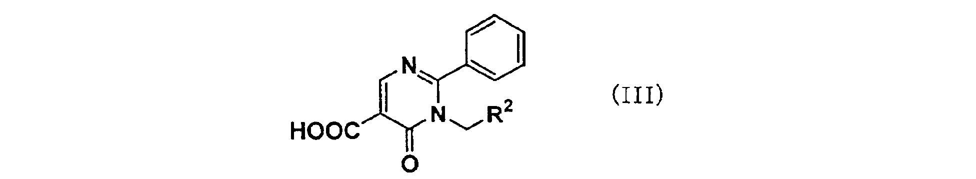 (wherein R² has the same meaning as described above) to an amidation.
   The amidation is a known reaction and examples include:
   1) a method of using an acid halide,
   2) a method of using a mixed acid anhydride,
   3) a method of using a condensing agent, and the like.

   1) The method of using an acid halide is carried out, for example, by reacting the compound represented by formula (III) with an acid halide (e.g., oxalyl chloride, thionyl chloride, or the like) in an inert organic solvent (e.g., chloroform, methylene chloride, diethyl ether, tetrahydrofuran, or the like) or without a solvent at -20°C to a refluxing temperature, and then reacting the resulting acid halide with a corresponding amine in the presence of a tertiary amine (e.g., pyridine, triethylamine, dimethylaniline, dimethylaminopyridine, or the like) in an inert organic solvent (e.g., chloroform, methylene chloride, diethyl ether, tetrahydrofuran, or the like) at -20°C to 40°C.
   2) The method of using a mixed acid anhydride is carried out, for example, by reacting the compound represented by formula (III) with an acid halide (e.g., pivaloyl chloride, tosyl chloride, mesyl chloride, or the like) or an acid derivative (e.g., ethyl chloroformate (ethyl chlorocarbonate), isobutyl chloroformate (isobutyl chlorocarbonate), or the like) in the presence of a tertiary amine (e.g., pyridine, triethylamine, dimethylaniline, dimethylaminopyridine, N-methylmorpholine, or the like) in an inert organic solvent (e.g., chloroform, methylene chloride, diethyl ether, tetrahydrofuran, or the like) or without a solvent at -20°C to 40°C, and then reacting the resulting mixed acid anhydride with a corresponding amine in an inert organic solvent (e.g., chloroform, methylene chloride, diethyl ether, tetrahydrofuran, or the like) at -20°C to 40°C.
   3) The method of using a condensing agent is carried out, for example, by reacting the compound represented by formula (III) with a corresponding amine in the presence or absence of a tertiary amine (e.g., pyridine, triethylamine, dimethylaniline, dimethylaminopyridine, or the like) in an organic solvent (e.g., chloroform, methylene chloride, dimethylformamide, diethyl ether, tetrahydrofuran, or the like) or without a solvent at 0°C to 40°C, using a condensing agent (e.g., 1,3-dicyclohexylcarb.odiimide (DCC), 1-ethyl-3-[3-(dimethylamino)propyl]carbodiimide (EDC), 1,1'-carbonyldiimidazole (CDI), 2-chloro-1-methylpyridinium iodide, or the like) with or without 1-hydroxybenzotriazole (HOBt).

   All of these reactions 1) to 3) are preferably carried out under an atmosphere of an inert gas (e.g., argon, nitrogen, or the like) under anhydrous conditions.
   As the method of the amidation for producing the compound represented by formula (I-3) from the compound represented by formula (III), the method of using a mixed acid anhydride is preferred.
(2) Among the compounds represented by formula (I), the compound wherein R¹ represents a CONHR³ group, i.e., the compound represented by formula (I-2): 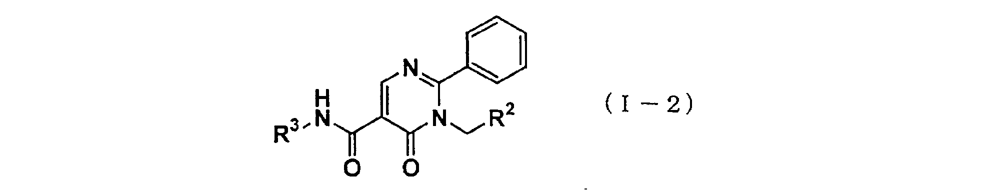 (wherein R³ represents a leaving group and other symbol has the same meaning as described above) can be produced by subjecting the compound represented by formula (I-3) described above to a leaving group-introducing reaction.
   The leaving group-introducing reaction for producing the compound represented by formula (I-2) can be carried out in accordance with the method described in T. W. Greene, *Protective Groups in Organic Synthesis,* Wiley, New York, 1991.
   For example, the reaction is carried out by reacting the compound represented by formula (I-3) with an acylating agent (e.g., acetic anhydride, acetyl chloride, pivaloyl chloride, or the like) or a sulfonating agent (e.g., mesyl chloride, tosyl chloride, or the like) in the presence of a base (e.g., triethylamine, pyridine, dimethylaminopyridine, or the like) in an inert organic solvent (e.g., chloroform, methylene chloride, tetrahydrofuran, dimethylformamide, or the like) at 0°C to 100°C.
(3) Among the compounds represented by formula (I), the compound wherein R¹ represents an amino group, i.e., the compound represented by formula (I-1): 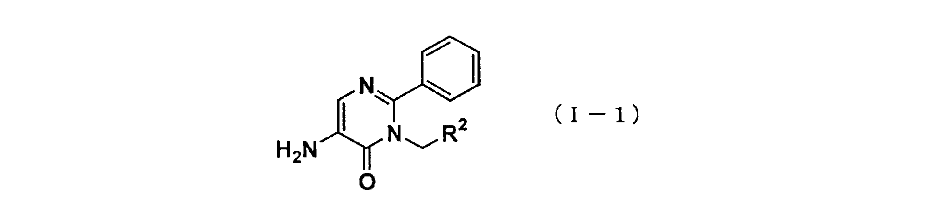 (wherein R² has the same meaning as described above) can be produced by subjecting the compound represented by formula (I-2) described above to a rearrangement. The rearrangement for producing the compound represented by formula (I-1) includes the Lossen rearrangement.

The Lossen rearrangement is carried out, for example, by reacting the compound represented by formula (I-2) in the presence of a base (e.g., 1,8-diazabicyclo[5.4.0]-7-undecene (DBU), or the like) in a mixed solvent of an inert organic solvent (e.g., chloroform, methylene chloride, tetrahydrofuran, dimethylformamide, or the like) and water at 0°C to a refluxing temperature.

Also, the compound represented by formula (II): 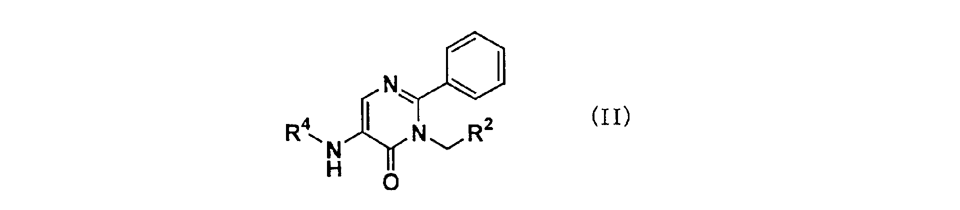 (wherein R⁴ represents a protective group of an amino group and other symbol has the same meaning as described above) can be produced by subjecting the compound represented by formula (I-1) to an amino group-protecting reaction.

The amino group-protecting reaction is known and can be carried out in accordance with the method described in T. W. Greene, *Protective Groups in Organic Synthesis,* Wiley, New York, 1991.

For example, the reaction is carried out by reacting the compound represented by formula (I-1) with a carbonic acid ester (or carbonate) derivative (e.g., methoxycarbonyl chloride, 9-fluorenylmethyloxycarbonyl chloride (Fmoc-Cl), benzyloxycarbonyl chloride (Cbz-CI), allyloxycarbonyl chloride, or the like) or a dicarbonate (e.g., di-t-butyl dicarbonate, dibenzyl dicarbonate, or the like) in the presence of a base (e.g., sodium carbonate, sodium hydrogen carbonate, potassium carbonate, sodium hydroxide, triethylamine, pyridine, diisopropylethylamine, dimethylaminopyridine, or the like) in a solvent (e.g., tetrahydrofuran, dioxane, chloroform, methylene chloride, acetonitrile, acetone, dimethylformamide, water, or a mixture thereof) at 0°C to a refluxing temperature.

The compound represented by formula (III) which is used as a starting material in the invention can be produced by or in accordance with the method described in EP528633 (JP-A-5-286946).

Other starting materials and each reagent in the invention are known *per se* or can be produced by known methods.

The product of each reaction in the present specification can be purified by a conventional purification means such as distillation under normal pressure or reduced pressure, high performance liquid chromatography using silica gel or magnesium silicate, thin layer chromatography, column chromatography, washing, recrystallization, or the like. The purification can be carried out at each reaction or after the completion of several reactions.

The compound represented by formula (I) can be converted into a salt by a known method. The salt is preferably non-toxic and water-soluble. Examples of suitable salts include salts of alkali metals (e.g., potassium, sodium, *etc.),* salts of alkaline earth metals (e.g., calcium, magnesium, *etc.),* ammonium salts, salts of pharmaceutically acceptable organic amines (e.g., tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, *etc.),* and the like.

The compound of the invention represented by formula (I) can be converted into a corresponding acid-adduct salt by a known method. The acid-adduct salt is preferably non-toxic and water-soluble. Examples of suitable acid-adduct salts include inorganic acid salts such as hydrochlorides, hydrobromides, sulfates, phosphates, nitrates and the like; and organic acid salts such as acetates, trifluoroacetates, lactates, tartrates, oxalates, fumarates, maleates, citrates, benzoates, methanesulfonates, ethanesulfonates, benzenesulfonates, toluenesulfonates, isethionates, glucuronates, gluconates and the like.

The compound of the invention represented by formula (I) or a non-toxic salt thereof described in the present specification can be converted into a hydrate by a known method.

### INDUSTRIAL APPLICABILITY

According to the invention, the compound represented by formula (II) useful as an intermediate for pharmaceuticals can be produced industrially and efficiently via the novel intermediate represented by formula (I).

### BEST MODE FOR CARRYING OUT THE INVENTION

The present invention will be explained below in detail with reference to Examples, but the invention is not limited thereto.

Solvents in parentheses shown in the parts of chromatographic separation and TLC are eluting solvents or developing solvents employed and ratios are by volume.

Solvents in parentheses shown in the parts of NMR are solvents employed in the measurement.

### Example 1

Synthesis of N-hydroxy-1-(2,2-dimethoxyethyl)-6-oxo-2-phenyl-1,6-dihydropyrimidin-5-ylcarboxamide (Compound represented by formula (I-3) wherein R² represents a dimethoxymethyl group):

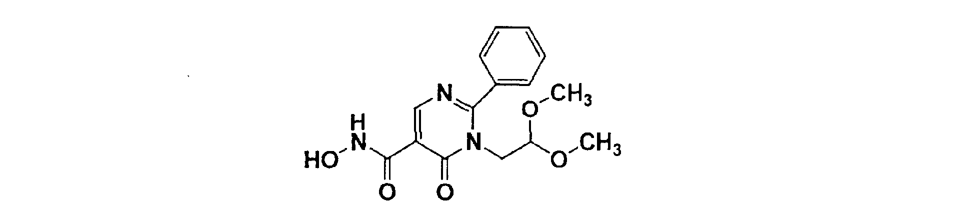

Under an argon atmosphere, 1-(2,2-dimethoxyethyl)-6-oxo-2-phenyl-1,6-dihydropyrimidin-5-ylcarboxylic acid (97.3 g) was dissolved in tetrahydrofuran (800 ml), and triethylamine (49.4 ml) was added thereto under ice-cooling. Then, isobutyl chlorocarbonate (45.6 ml) was added dropwise thereto at 5°C or lower, followed by stirring at 5°C or lower for 1 hour. To the reaction mixture was added a 50% aqueous hydroxylamine solution (422 ml), followed by stirring for 20 minutes. Water was added to the reaction mixture, followed by extraction with ethyl acetate (200 ml). The organic layer was washed with saturated saline (200 ml), followed by concentration. Toluene (200 ml) was added to the residue, followed by concentration. Isopropyl ether (1000 ml) was added to the crude crystals, followed by heating under reflux for 10 minutes to wash the crystals. Thereafter, the mixture was cooled to room temperature and the crystals were collected by filtration. The resulting crystals were dried at room temperature under reduced pressure overnight to obtain the compound of the invention having the following physical properties (93.3 g, 91% yield).
TLC: Rf 0.5 (ethyl acetate);
NMR (CDCl₃): δ 8.95 (s, 1H), 7.62-7.45 (m, 5H), 4.76 (t, J=5.8 Hz, 1H), 4.21 (2H, d, J=5.8 Hz), 3.30 (6H, s).

### Example 2

Synthesis of N-acetyloxy-1-(2,2-dimethoxyethyl)-6-oxo-2-phenyl-1,6-dihydropyrimidin-5-ylcarboxamide (Compound represented by formula (I-2) wherein R² represents a dimethoxymethyl group):

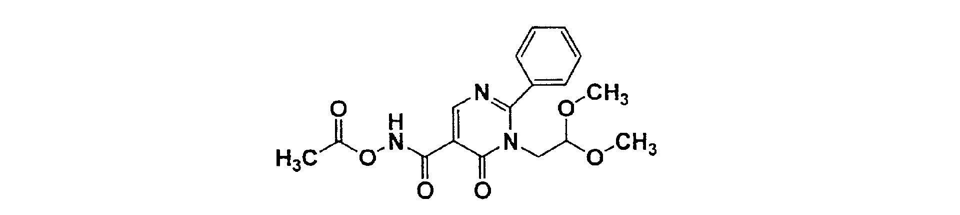

Under an argon atmosphere, pyridine (5.66 ml) was added to a tetrahydrofuran (50 ml) suspension of the compound produced in Example 1 (15.95 g), and acetic anhydride (5.19 ml) was further added dropwise thereto, followed by stirring for 20 minutes. Ethyl acetate (150 ml) and 1N hydrochloric acid (84 ml) were added to the reaction mixture and the resulting layers were separated. The organic layer was washed with saline (a mixture of saturated saline (50 ml) and water (50 ml)) and a mixed solution of saturated saline (50 ml) and a saturated aqueous sodium hydrogen carbonate solution (50 ml), successively, followed by concentration to obtain the compound of the invention having the following physical properties (16.9 g, crude product).
TLC: Rf 0.44 (toluene:acetone = 4:1);
NMR (CDCl₃): δ 8.99 (s, 1H), 7.62-7.45 (m, 5H), 4.79 (t, J=5.4 Hz, 1H), 4.22 (d, J=5.4 Hz, 2H), 3.30 (s, 6H), 2.30 (s, 3H).

### Example 3

Synthesis of 5-amino-1-(2,2-dimethoxyethyl)-6-oxo-2-phenyl-1,6-dihydropyrimidine (Compound represented by formula (I-1) wherein R² represents a dimethoxymethyl group):

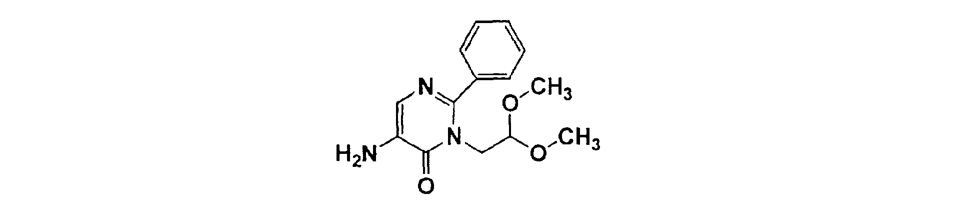

A tetrahydrofuran (200 ml) suspension of the compound produced in Example 2 (16.9 g) was heated to obtain a solution. Then, water (18 ml) was added thereto at 40°C and 1,8-diazabicyclo[5.4.0]-7-undecene (9.35 ml) was added thereto at 50°C, followed by heating under reflux for 1 hour. The reaction mixture was cooled to room temperature and then toluene (100 ml) and a saturated aqueous ammonium chloride solution (100 ml) were added thereto, and the resulting layers were separated. The organic layer was washed with a saturated aqueous ammonium chloride solution and saturated saline (100 ml), successively, followed by concentration to obtain the compound of the invention having the following physical properties (15.7 g, crude product).
TLC: Rf 0.26 (toluene:acetone = 4:1);
NMR (CDCl₃): δ 7.55-7.38 (m, 6H), 4.77 (t, J=5.6 Hz, 1H), 4.14 (d, J=5.6 Hz, 2H), 4.02 (brs, 2H), 3.27 (s, 6H).

### Example 4

Synthesis of 5-benzyloxycarbonylamino-1-(2,2-dimethoxyethyl)-6-oxo-2-phenyl-1,6-dihydropyrimidine (Compound represented by formula (II) wherein R² represents a dimethoxymethyl group and R⁴ represents a benzyloxycarbonyl group):

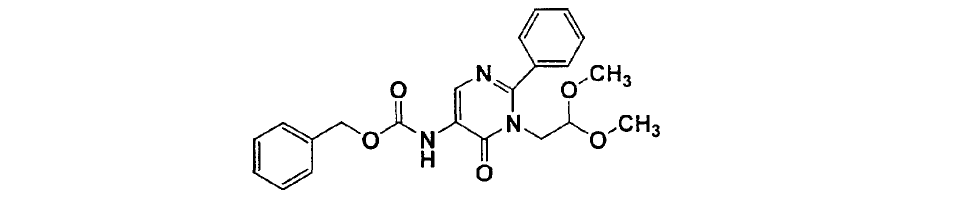

A mixture of the compound produced in Example 3 (15.7 g), tetrahydrofuran (75 ml), water (75 ml), and sodium hydrogen carbonate (5.88 g) was ice-cooled, benzyloxycarbonyl chloride (8.56 ml) was added dropwise thereto at 5°C or lower, followed by stirring at 5°C overnight. The reaction mixture was extracted with ethyl acetate (100 ml). The organic layer was washed with saturated saline (50 ml), followed by concentration to obtain the compound of the invention having the following physical properties (22.0 g, crude product).
TLC: Rf 0.67 (toluene:acetone = 4:1);
NMR (CDCl₃): δ 8.73 (brs, 1H), 7.60-7.30 (m, 10H), 5.24 (s, 2H), 4.71 (t, J=5.5 Hz, 1H), 4.15 (d, J=5.5 Hz, 2H), 3.26 (s, 6H).

## Claims

1. A compound represented by formula (I); 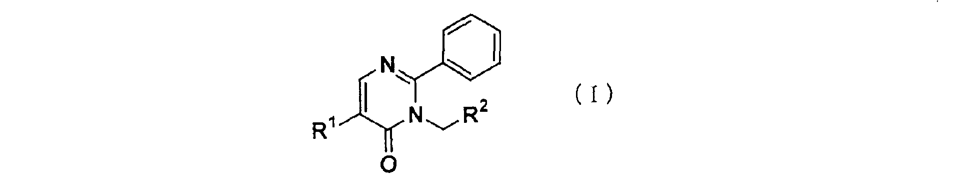 (wherein R¹ represents a 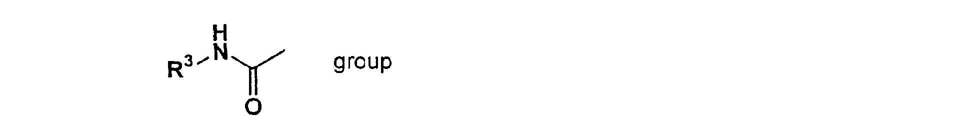 (in the group, R³ represents a leaving group), a hydroxycarbamoyl group, or an amino group; and R² represents a group capable of converting into a formyl group), a non-toxic salt thereof, or a hydrate thereof.

2. The compound according to claim 1, wherein R² is a dimethoxymethyl group, a diethoxymethyl group, or a vinyl group in formula (I).

3. The compound according to claim 1, wherein R¹ is an amino group in formula (I), said compound being represented by formula (I-1): 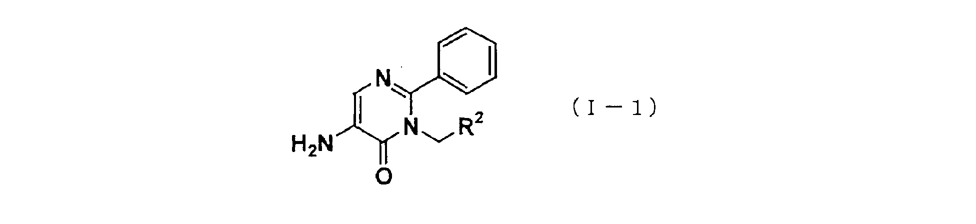 (wherein R² has the same meaning as in claim 1).

4. The compound according to claim 1, wherein R¹ is a CONHR³ group (in the group, R³ has the same meaning as in claim 1) in formula (I), said compound being represented by formula (I-2): 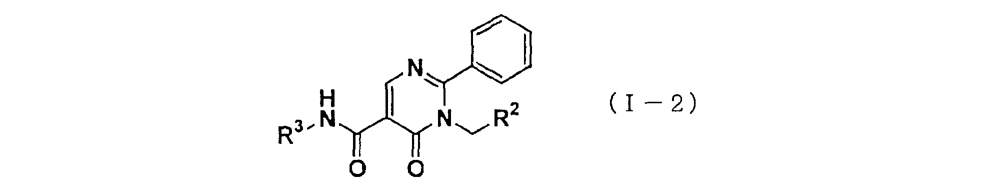 (wherein R² and R³ have the same meanings as in claim 1).

5. The compound according to claim 4, wherein R³ is an acetyloxy group, a pivaloyloxy group, a benzoyloxy group, a mesyl group, or a tosyl group.

6. The compound according to claim 1, wherein R¹ is a hydroxycarbamoyl group in formula (I), said compound being represented by formula (I-3): 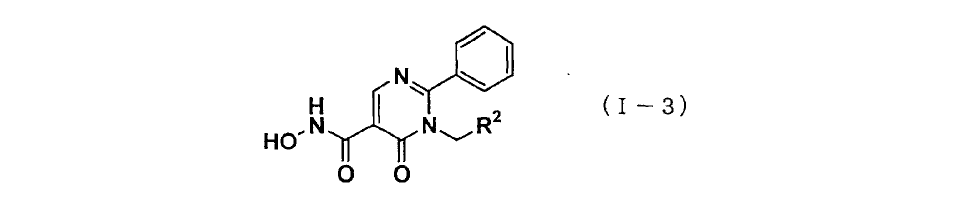 (wherein R² has the same meaning as in claim 1).

7. The compound according to claim 3, wherein the compound is 5-amino-1-(2,2-dimethoxyethyl)-6-oxo-2-phenyl-1,6-dihydropyrimidine.

8. The compound according to claim 4, wherein the compound is N-acetyloxy-1-(2,2-dimethoxyethyl)-6-oxo-2-phenyl-1,6-dihydropyrimidin-5-ylcarboxamide.

9. The compound according to claim 6, wherein the compound is N-hydroxy-1-(2,2-dimethoxyethyl)-6-oxo-2-phenyl-1,6-dihydropyrimidin-5-ylcarboxamide.

10. A process for producing a compound represented by formula (I-3): 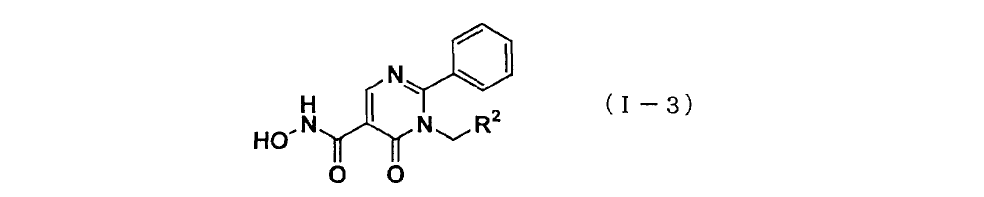 (wherein R² has the same meaning as in claim 1), comprising subjecting a compound represented by formula (III): 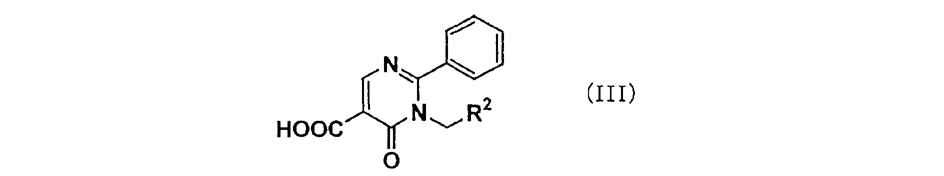 (wherein R² has the same meaning as described above) to an amidation.

11. A process for producing a compound represented by formula (I-2): 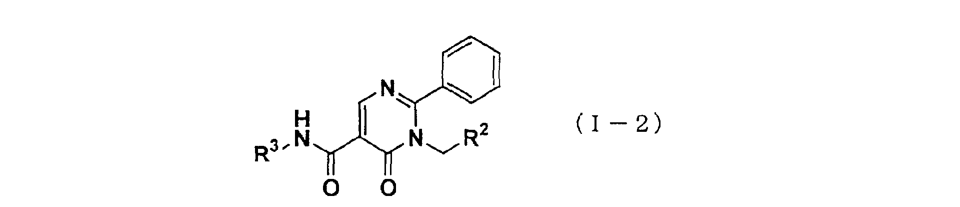 (wherein R² and R³ have the same meanings as in claim 4), comprising subjecting a compound represented by formula (I-3): 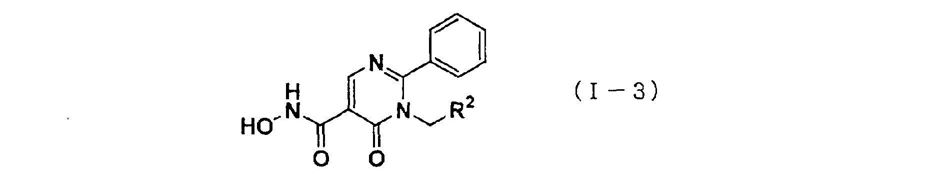 (wherein R² has the same meaning as in claim 6) to a leaving group-introducing reaction.

12. A process for producing a compound represented by formula (I-1): 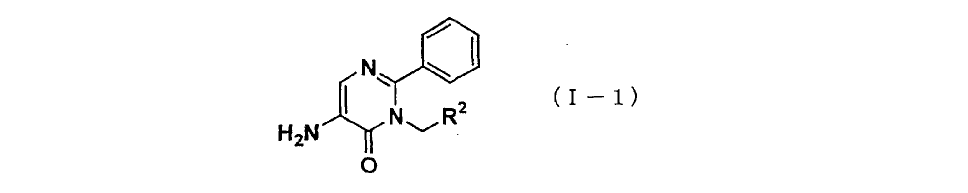 (wherein R² has the same meaning as described above), comprising subjecting a compound represented by formula (I-2): 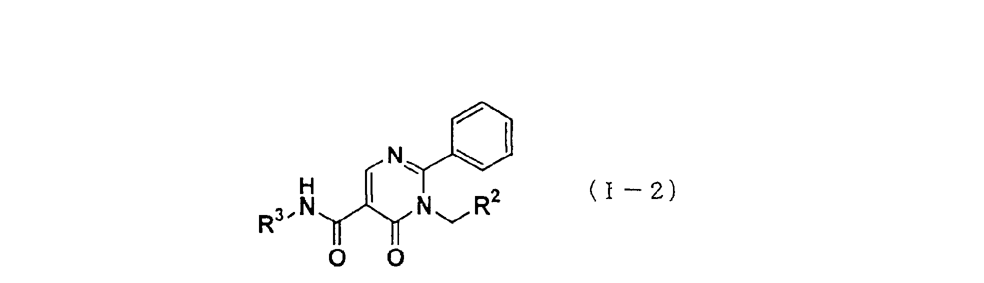 (wherein R² and R³ have the same meanings as described above) to a rearrangement.

13. A process for producing a compound represented by formula (II): 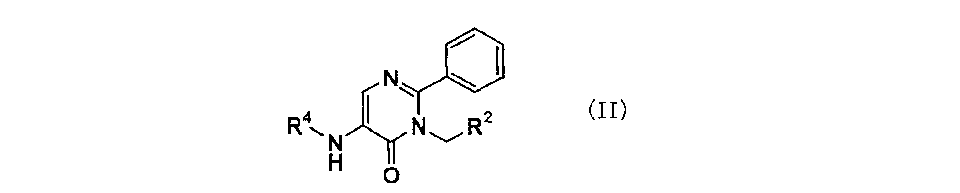 (wherein R⁴ represents a protective group of an amino group; and R² has the same meaning as in claim 3), comprising subjecting a compound represented by formula (I-1): 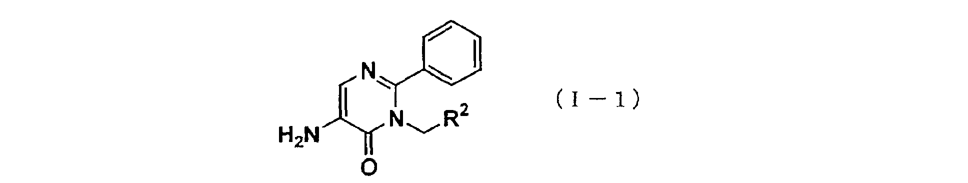 (wherein R² has the same meaning as described above) to an amino group-protecting reaction.
